(19) Europäisches Patentamt
European Patent Office
Office européen des brevets

(11) Publication number: **0 068 715**
**A1**

(12) # EUROPEAN PATENT APPLICATION

(21) Application number: **82303112.5**

(22) Date of filing: **16.06.82**

(51) Int. Cl.³: **C 07 D 498/04**, A 61 K 31/42
//
(C07D498/04, 263/00, 205/00), (A61K31/42, 31/43),(A61K31/42, 31/545)

(30) Priority: **30.06.81 GB 8120166**

(43) Date of publication of application: **05.01.83**
**Bulletin 83/1**

(84) Designated Contracting States: **BE CH DE FR GB IT LI NL SE**

(71) Applicant: **BEECHAM GROUP PLC, Beecham House Great West Road, Brentford Middlesex (GB)**

(72) Inventor: **Gilpin, Martin Leonard, 42 Reeve Road, Reigate Surrey (GB)**

(74) Representative: **Hesketh, Alan, Dr. et al, European Patent Attorney Beecham Pharmaceuticals Great Burgh Yew Tree Bottom Road, Epsom, Surrey, KT18 5XQ (GB)**

(54) **Beta-lactam antibacterial agents, their preparation and use.**

(57) Compounds of the formula (I):

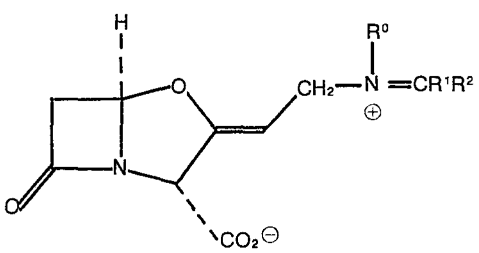

(I)

and acid addition salts and salted esters thereof; wherein $R^0$ is an optionally substituted $C_{1-12}$ hydrocarbon group;
$R^1$ is a group $NR^3R^4$;
$R^2$ is hydrogen or a group R, or $NR^5R^6$;
R is an optionally substituted hydrocarbon or heterocyclic group;
$R^3$ is hydrogen or a group R or OR;
$R^4$, $R^5$ and $R^6$ are independently hydrogen or a group R; or
$R^3$ and $R^4$, $R^5$ and $R^6$, or $R^1$ and $R^2$ may be joined to form a heterocyclic ring.

EP 0 068 715 A1

0068715

## β-Lactam Antibacterial Agents, Their Preparation and Use

This invention relates to β-lactam antibacterial agents, to processes for their preparation and to compositions containing them. In particular this invention relates to a novel class of amidine derivatives of clavulanic acid that have good antibacterial and β-lactamase inhibitory qualities.

Accordingly the present invention provides the compounds of the formula (I):

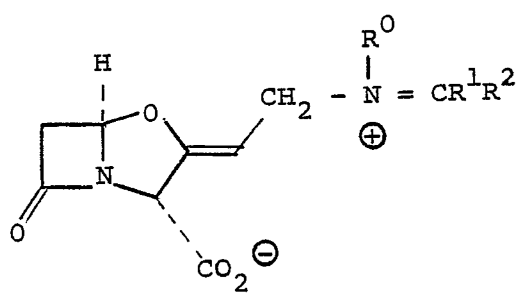

(I)

and acid addition salts and salted esters thereof; wherein $R^0$ is an optionally substituted $C_{1-12}$ hydrocarbon group;

$R^1$ is a group $NR^3R^4$;

$R^2$ is hydrogen or a group R, or $NR^5R^6$;

R is an optionally substituted hydrocarbon or heterocyclic group;

$R^3$ is hydrogen or a group R or OR;

$R^4$, $R^5$ and $R^6$ are independently hydrogen or a group R; or

$R^3$ and $R^4$, $R^5$ and $R^6$, or $R^1$ and $R^2$ may be joined to form a heterocyclic ring.

The group R may represent optionally substituted $C_{1-10}$alkyl, $C_{2-10}$alkenyl, $C_{2-10}$alkynyl, $C_{3-10}$cycloalkyl, phenyl, or heterocyclic.

When the group R, and thus the groups $R^3$, $R^4$, $R^5$ and $R^6$, are substituted,suitable substituents include $C_{1-6}$alkyl, phenyl, heterocyclic, $C_{3-10}$ cycloalkyl, $C_{1-6}$ alkoxy, $C_{1-6}$alkanoyl, oxo, $C_{1-6}$alkanoyloxy, chloro, bromo, fluoro, mercapto, $C_{1-6}$alkylthio, nitro, amino, $C_{1-6}$ alkylamino, di-$C_{1-6}$alkylamino, hydroxy, carboxy or pharmaceutically acceptable salt or ester thereof, and cyano.

In particular, for example, the group $R^3$ may represent hydrogen, $C_{1-10}$alkyl, $C_{1-10}$alkoxy, $C_{2-10}$alkenyl, $C_{2-10}$alkynyl, $C_{3-10}$cycloalkyl, $C_{4-12}$cycloalkylalkyl, phenyl, phenyl($C_{1-6}$)alkyl, heterocyclyl, heterocyclyl ($C_{1-6}$)alkyl or phenyl($C_{1-6}$)alkoxy; and $R^4$, $R^5$ and $R^6$ may represent independently hydrogen, $C_{1-10}$ alkyl, $C_{2-10}$ alkenyl, $C_{2-10}$alkynyl, $C_{3-10}$ cycloalkyl, $C_{4-12}$ cycloalkylalkyl, phenyl, phenyl($C_{1-6}$)alkyl, heterocyclyl and heterocyclyl($C_{1-6}$)alkyl; any of the above $R^3$, $R^4$, $R^5$ and $R^6$ groups optionally being substituted.

When used herein the term "heterocyclyl" means a monocyclic or bicyclic ring system comprising 4 to 10 ring atoms, one, two or three of which are selected from oxygen, sulphur or nitrogen.

Suitably $R^3$, $R^4$, $R^5$ and $R^6$ are independently selected from the group consisting of hydrogen, $C_{1-6}$ alkyl, $C_{3-10}$ alkenyl, phenyl, phenylalkyl and heteroaralkyl, any of the above groups optionally being substituted by hydroxy, $C_{1-3}$ alkoxy, $C_{1-3}$ alkyl, halo, $C_{1-3}$ alkanoyloxy, or carboxy, or a pharmaceutically acceptable salt or $C_{1-4}$

alkyl ester or benzyl, nitrobenzyl, $C_{1-3}$ alkylbenzyl, $C_{1-3}$ alkoxybenzyl, halobenzyl or $C_{1-3}$ alkoxycarbonylbenzyl ester thereof.

Preferably $R^3$ and $R^5$ are selected from hydrogen, $C_{1-6}$ alkyl, phenyl, benzyl, $C_{1-3}$ alkoxybenzyl, $C_{1-3}$ alkylbenzyl, nitrobenzyl, chlorobenzyl, fluorobenzyl, bromobenzyl, alkoxy $C_{1-6}$ alkyl, carboxyalkyl or a pharmaceutically acceptable salt or $C_{1-4}$ alkyl ester or benzyl or nitrobenzyl ester thereof, $C_{1-3}$ alkoxycarbonylbenzyl or hydroxy $C_{1-6}$ alkyl.

Preferably $R^4$ and $R^6$ are selected from hydrogen, $C_{1-6}$ alkyl or benzyl.

Suitably $R^3$ is a $C_{1-10}$ alkoxy or phenyl($C_{1-6}$) alkoxy group, for example, methoxy, ethoxy, propoxy, butoxy or benzyloxy.

Suitably $R^0$ is a $C_{1-6}$ alkyl group optionally substituted by hydroxy, carboxy or salts or esters thereof, cyano, a hetercyclyl ring bonded _via_ a nitrogen atom, or a group of the sub-formula i):

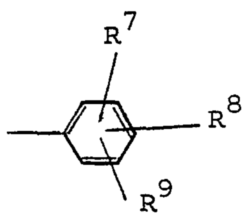

(i)

wherein $R^7$ is a hydrogen, fluorine, chlorine or bromine atom or a $C_{1-3}$ alkyl, $C_{1-3}$ alkoxy, $C_{1-3}$ alkanoyloxy, hydroxy, $C_{1-4}$ alkoxycarbonyl group, or a group $-N(R^{10})COR^{11}$, $-N(R^{10})SO_2R^{11}$ or $-CONR^{10}R^{11}$ wherein $R^{10}$ is a hydrogen atom, $C_{1-3}$ alkyl, phenyl or benzyl and $R^{11}$ is $C_{1-3}$ alkyl, phenyl or benzyl; $R^8$ is a hydrogen, fluorine or chlorine atom or is $C_{1-3}$ alkyl, $C_{1-3}$ alkoxy or $C_{1-3}$ alkanoyloxy; and $R^9$ is a hydrogen, fluorine or chlorine atom or a $C_{1-3}$ alkoxy or alkyl group. Suitably also $R^0$ is a $C_{3-9}$ cycloalkyl group,

for example cyclopropyl, cyclobutyl, cyclopentyl and cyclohexyl, of these cyclopropyl is favoured.

Suitably also $R^0$ is an optionally substituted heteroarylmethyl group wherein the heteroaryl moiety is an aromatic monocyclic or bicyclic heterocyclic group bonded via a carbon atom. Suitable aromatic monocyclic heterocyclic groups are those containing 5 or 6 ring atoms. Suitable aromatic bicylic heterocyclic groups are those having a benzene ring fused to an aromatic heterocyclic group containing 5 or 6 atoms.

More suitably $R^0$ is a $C_{1-6}$ alkyl group optionally substituted by hydroxy, carboxy or salts or esters thereof or cyano, for example $R^0$ may be $C_{1-6}$ alkyl either branched or straight-chain such as methyl, ethyl, n-propyl, isopropyl, n-butyl, sec-butyl, isobutyl, pentyl or hexyl, of these ethyl, n-propyl, and isobutyl are favoured with the isobutyl being preferred; alternatively $R^0$ may be $C_{1-6}$ alkyl substituted by carboxy, for example carboxyethyl, carboxypropyl and carboxybutyl, or $R^0$ may be $C_{1-6}$ alkyl substituted by cyano such as cyanoethyl, or $R^0$ may be $C_{1-6}$ alkyl substituted by hydroxy such as hydroxyethyl.

Suitably also $R^0$ is a $C_{1-6}$ alkyl group, preferably a methyl group substituted by a group of the sub-formula i) as hereinbefore defined. Thus favoured groups $R^0$ include benzyl and benzyl substituted by one, two or three groups or atoms selected from fluorine, chlorine, bromo, $C_{1-3}$ alkyl, $C_{1-3}$ alkoxy, hydroxy, $C_{1-6}$ alkanoylamino such as acetamido, and $C_{1-4}$ alkoxycarbonyl such as methoxy-carbonyl. Preferably $R^0$ is benzyl.

One class of compounds of this invention are amidines of the fomula (II):

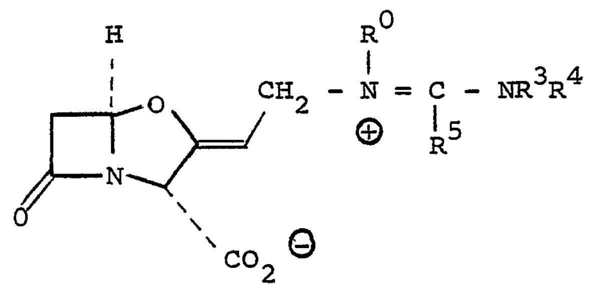

(II)

and acid addition salts and salted esters thereof wherein $R^0$,$R^3$, $R^4$ and $R^5$ are as defined hereinbefore.

Preferably in the compounds of the formula (II) $R^3$, $R^4$ and $R^5$ are independently selected from the group consisting of hydrogen, $C_{1-6}$ alkyl such as methyl, ethyl, propyl and butyl, phenyl and benzyl. Preferably $R^0$ is benzyl or $C_{1-6}$ alkyl such as isobutyl.

In an alternative aspect of the compounds of the formula (II) $R^3$ and $R^4$ may be joined to form (together with the nitrogen atom to which they are attached) a heterocyclyl ring, for example a pyrrolidino, morpholino, piperidino or azepino ring. In a further aspect either $R^3$ or $R^4$ is joined to $R^5$ to form (together with the nitrogen and carbon atoms to which they are attached) a heterocyclyl ring, for example a pyrrolidinyl, morpholinyl, piperidinyl or azepinyl ring.

In another aspect of this invention there are provided guanidines of the formula(III):

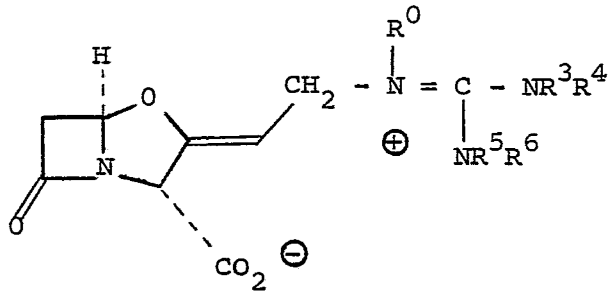

(III)

and acid addition salts and salted esters thereof, wherein $R^0$, $R^3$, $R^4$, $R^5$ and $R^6$ are as defined hereinbefore.

Preferably in the compounds of the formula (III) $R^3$, $R^4$, $R^5$ and $R^6$ are independently selected from the group consisting of hydrogen, $C_{1-6}$ alkyl such as methyl, ethyl, propyl and butyl, phenyl and benzyl. Preferably $R^0$ is benzyl or $C_{1-6}$ alkyl such as isobutyl,

In a further aspect of this invention there are provided pseudoureas and pseudothioureas of the formula (IV):

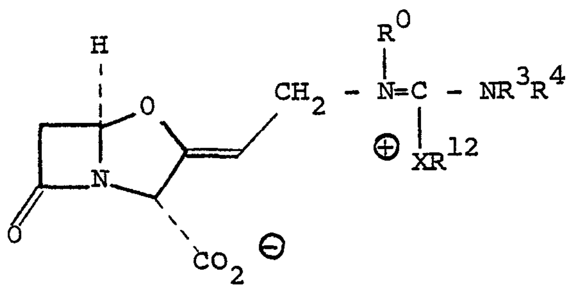

(IV)

and acid addition salts and salted esters thereof, wherein $R^0$, $R^3$ and $R^4$ are as hereinbefore defined, X is an oxygen or sulphur atom, and $R^{12}$ is of the value $R^5$ except that it is not a hydrogen atom.

Preferably in the compounds of the formula (IV) $R^3$ is selected from the group consisting of phenyl, benzyl and $C_{1-6}$ alkyl such as methyl, ethyl, propyl and butyl. Preferably $R^4$ is a hydrogen atom. Preferably $R^0$ is benzyl or $C_{1-6}$ alkyl such as isobutyl.

It is to be realised that the compounds of the formulae (II) - (IV) may also be represented in the form of formula (V):

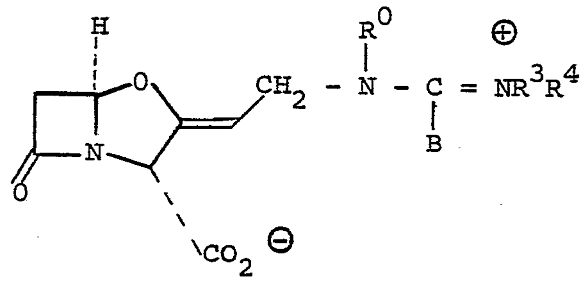

(V)

wherein B represents $R^5$, $NR^5R^6$ or $XR^{12}$, wherein $R^0$, $R^3$, $R^4$, $R^5$, $R^6$, $R^{12}$ and X are as hereinbefore defined.

The major utility of the compounds of the formulae (I) - (V) and acid addition salts and salted esters thereof is as pharmaceuticals, and accordingly the salts and esters of the compounds of this invention are preferably pharmaceutically acceptable. The compounds of this invention both pharmaceutically acceptable and non-pharmaceutically acceptable may be used also as antibacterial agents or β-lactamase inhibitors in non-pharmaceutical usage such as a disinfectant or paint additive.

Suitable pharmaceutically acceptable salting-ions for use in salifying any carboxy group present in the groups $R^3$, $R^4$, $R^5$ and $R^6$ include alkali and alkaline earth metal salts such as the sodium, potassium, calcium and magnesium salts.

The compounds of this invention may be presented in the form of their acid addition salts if desired. The acid used to form the salt will most suitably be pharmaceutically acceptable, but non-pharmacuetically acceptable acid addition salts are also envisaged, for example as intermediates in the preparation of the pharmaceutically acceptable salts by ion-exchange. Suitable pharmaceutically acceptable acid addition salts include those of inorganic and organic acids, such as hydrochloric, phosphoric, sulphuric, methanesulphonic, toluenesulphonic, citric, malic, acetic, lactic, tartaric, propionic and succinic acids.

Most suitably the acid addition salt is provided as a solid and preferably as a crystalline solid.

By "salted ester" we mean that the carboxy group attached to the clavulanic acid ring system is esterified, and as the positively charged nitrogen containing group requires a counter-balancing ion, an anion is provided. Suitably this anion is a halide ion for example chloride.

Suitable esters of the compounds of this invention include those cleavable by biological methods such as enzymatic hydrolysis, in-vivo hydrolysis, and those cleavable by chemical methods such as hydrogenolysis, hydrolysis, electrolysis or photolysis.

Preferably any ester group is pharmaceutically acceptable, for example in-vivo hydrolysable. In-vivo hydrolysable esters are those esters which hydrolyse in the human body to produce the parent acid or its salt. Such esters may be identified by administration to a test animal such as a rat or a mouse by intravenous administration and thereafter examining the test animal's body fluids for the presence of the compound of the formula (I) or its salt.

Suitable esters of this type include those of sub-formula a):

$$- CO_2 - CHA^1 - OCOA^2 \qquad (a)$$

wherein $A^1$ is a hydrogen atom or a methyl group; and $A^2$ is a $C_{1-4}$ alkyl, $C_{1-4}$ alkoxy or phenyl group, or $A^1$ and $A^2$ are joined to form a phthalidyl, dimethylphthalidyl or dimethoxyphthalidyl group. Preferred groups include the acetoxymethyl, α-acetoxyethyl, pivaloyloxymethyl, α-ethoxycarbonyloxyethyl, ethoxycarbonyloxymethyl, dimethoxyphthalidyl and phthalidyl groups. Further suitable esters include di($C_{1-6}$) alkylamino($C_{1-6}$) alkyl esters such as dimethylaminomethyl, dimethylaminoethyl, diethylaminomethyl and diethylaminoethyl.

The present invention provides a pharmaceutical composition which comprises a compound of this invention and a pharmaceutically acceptable carrier.

The compositions of the invention include those in a form adapted for oral, topical or parenteral use and may be used for the treatment of the infection in animals for example mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating anti-biotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form.

The injectable solution of the compound of this invention may be made up in a sterile pyrogen-free liquid such as water, aqueous ethanol or the like.

An alternative approach to administering the compounds of this invention is to utilise an injectable suspension. Such suspensions may be made up in sterile water; sterile saline or the like, and may also contain suspending agents such as polyvinylpyrrolidone, lecithin or the like. Alternatively such compositions may be prepared in an acceptable oil suspending agent such as arachis oil or its equivalent. For use in such suspensions the compounds of this invention should be in the form of find particles.

Unit dose compositions comprising a compound of this invention adapted for oral administration form a further suitable composition aspect of this invention.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compound of the formula may be present in the composition as sole therapeutic agent or it may be present together with other therapeutic agents such as a penicillin or cephalosporin. Considerable advantages accrue from the inclusion of a penicillin or cephalosporin which shows instability to β-lactamases since the resulting composition shows enhanced effectiveness (synergy). Suitable penicillins, cephalosporins or other β-lactam anti-biotics for inclusion in such synergistic compositions include not only those

known to be highly susceptible to β-lactamases but also those which have a degree of intrinsic resistance to β-lactamases.

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, celbenicillin, pipericillin, and other known penicillins including pro-drugs therefor such as their in vivo hydrolysable esters such as the acetoxymethyl, pivaloyloxymethyl, α-ethoxycarbonyloxyethyl or phthalidyl esters of ampicillin, benzylpenicillin or amoxycillin, and aldehyde or ketone adducts of penicillins containing a 6-α-aminoacetamide side chain (such as hetacillin, metampicillin and analogous derivatives of amoxycillin) or α-esters of carbenicillin or ticarcillin such as their phenyl or indanyl α-esters.

Suitable cephalosporins for inclusion in the compositions of this invention include cefatrizine, cephaloridine, cephalothin, cefazolin, cephalexin, cephacetrile, cephamandole nafate, cephapirin, cephradine, 4-hydroxycephalexin, cefaparole, cephaloglycin, cefoperazone and other known cephalosporins or pro-drugs thereof.

Such compounds are frequently used in the form of a salt or hydrate of the like.

Naturally if the penicillin or caphalosporin present in the composition is not suitable for oral administration then the composition will be adapted for parenteral administration.

Highly favoured penicillins for use in the compositions of this invention include ampicillin,amoxycillin, carbenicillin and ticarcillin. Such penicillins may be used as a pharmaceutically acceptable salt such as the sodium salt. Alternatively the ampicillin or amoxycillin may be used in the form of find particles of the zwitterionic form (generally as ampicillin trihydrate or amoxycillin trihydrate) for use in an injectable suspension, for example in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin which may be in the form of a pharmaceutically acceptable salt for example the sodium salt.

When present together with a cephalosporin or penicillin, the ratio of a compound of the invention to the penicillin or cephalosporin agent may vary over a wide range of ratios, such as from 10:1 to 1:10 for example about 3:1, 2:1, 1:1, 1:2, 1:3, 1:4, 1:5 or 1:6, (wt/wt, based on pure free anti-biotic equivalent). Orally administrable compositions containing a compound of the invention will normally contain relatively more synergist than corresponding injectable compositions.

The total quantity of a compound of the invention in any unit dosage form will normally be between 25 and 1000 mg and will usually be between 50 and 500 mg, for example about 62.5, 100, 125, 150, 200 or 250 mg.

Compositions of this invention may be used for the treatment of infections of <u>inter alia</u> the respiratory tract, the urinary tract and soft tissues in humans and mastitis in cattle.

Normally between 50 and 3000 mg of the compounds of the invention will be administered each day of treatemnt but more usually between 100 and 1000 mg of the compounds of the invention will be administered per day, for example at 1-6 doses, more usually as 2, 3 or 4 doses. However, for the treatment of more severe systemic infections or infections of particularly intransigent organisms higher doses may be used in accordance with clinical practice.

The penicillin or cephalosporin in the synergistic composition of this invention will normally be present at approximately the amount at which it is conventionally used which will usually be expected to be from about 62.5 to 3000 mg per dose, more usually about 125, 250, 500 or 1000 mg per dose.

One particularly favoured composition of this invention will contain from 150 to 1000 mg of amoxycillin as the trihydrate or sodium salt and from 25 to 500 mg of a compound of this invention.

A further particularly favoured composition of this invention will contain from 150 to 1000 mg of ampicillin or a pro-drug therefor and from 25 to 500 mg of a compound of this invention.

Most suitably this form of composition will contain ampicillin trihydrate, ampicillin anhydrate, sodium ampicillin, hetacillin, pivampicillinhydrochloride,

bacampicillin hydrochloride, or talampicillin hydrochloride. Most suitably this form of the composition will contain a compound of this invention when in crystalline form.

Most suitably the preceding composition will contain from 200 to 700 mg of the penicillin component. Most suitably the preceding composition will comprise from 50 to 250 mg of a compound of this invention preferably in crystalline form.

Such compositions may be adapted for oral or parenteral use except when containing an in vivo hydrolysable ester of ampicillin or amoxycillin in which case the compositions will not be adapted for parenteral administration.

Another particularly favoured composition of this invention will contain from 200 to 2000 mg of carbenicillin, ticarcillin or a pro-drug therefor and from 50 to 500 mg of a compound of the invention.

Suitably this form of composition will contain di-sodium carbenicillin. Suitably this form of the composition will contain di-sodium ticarcillin.

More suitably this form of the composition will contain from 75 to 250 mg of a compound of this invention preferably in crystalline form. Such compositions containing di-salts of carbenicillin and ticarcillin will be adapted for parenteral administration.

The present invention also provides a method of treating bacterial infections in animals such as humans or domestic mammals which comprises the administration of a compositions of this invention.

Commonly the infection treated will be due to a strain of <u>Staphylococcus aureus</u>, <u>Klebsiella aerogenes</u>, <u>Escherichia coli</u>, <u>Proteus sp.</u>, <u>Bacteroides fragilis</u> or the like. The organisms believed to be most readily treated by an antibacterially effective amount of a compound of this invention is <u>Staphylococcus aureus</u>. The other organisms named are more readily treated by using a synergistically effective amount of the compound of the invention and a penicillin or cephalosporin. The administration of the two components may take place separately but in general we prefer to use a composition containing both the synergist and the penicillin or cephalosporin.

The indications for treatment include respiratory tract and urinary tract infections in humans and mastitis in cattle.

In a further aspect the present invention provides a process for the preparation of the compounds of the formulae (II) and (III) and acid addition salts and salted esters thereof, which process comprises the reaction of a compound of the formula (VI) or a derivative thereof that allows alkylation to occur:

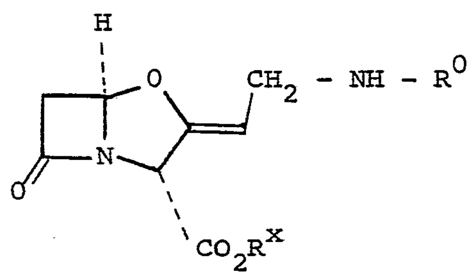

(VI)

wherein $R^0$ is as hereinbefore defined and $R^x$ is a hydrogen atom or a carboxy-blocking group; with either the compound of the formula (VII) or (VII):

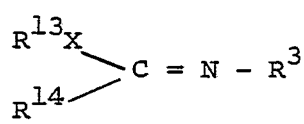

(VII)

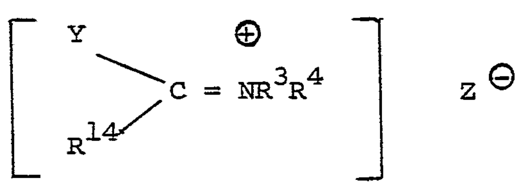

(VII)

wherein $R^{13}$ is $C_{1-6}$ alkyl, $R^{14}$ is a group $R^5$ or $NR^5R^6$ wherein $R^5$ and $R^6$ are as hereinbefore defined, Y is halo or $C_{1-6}$ alkoxy, X is an oxygen or sulphur atom, and $Z^-$ is a salting-ion, and $R^3$ and $R^4$ are as defined hereinbefore; and thereafter if necessary

i) removing the blocking group,

ii) forming an acid addition salt,

iii) forming a salted ester.

The compounds of the formula (VI) wherein $R^x$ is hydrogen may suitably be in the form of a zwitterion. The compound of the formula (VII) is preferably used in the form of its acid addition salt, for example as the hydrochloride.

An example of a derivative that allows alkylation to occur is a silyl derivative, for example, the trimethyl.

Suitably $R^{13}$ is a methyl or ethyl group. Suitably Y is a chloro atom. Suitably also Y is $C_{1-6}$ alkoxy such as methoxy or ethoxy. Suitably Z is halo such as chloro, or is tetrafluoroborate.

Reaction of the compound of the formula (VI) with either the compound of the formula (VII) or (VIII) is suitably performed in water, ether, dioxan, tetrahydrofuran, dimethylformamide, dichloromethane or mixtures of such solvents. Suitably the reaction is performed at elevated temperature, for example from 30°C to 50°C. Suitably the reaction is carried out at the reflux temperature of a suitable solvent such as dichloromethane. In aqueous media we have found it convenient to carry out the reaction at a basic pH, for example 7.5 to 10, preferably 8 to 9.

However, when Y is $C_{1-6}$ alkoxy in the compound of the formula(VIII) the reaction is suitably performed in an inert organic solvent, such as a chlorinated solvent, for example dichloromethane or chloroform; using approximately one equivalent of an organic base of low nucleophilicity, for example diazabicyclononene.

In another aspect the present invention provides a process for the preparation of compounds of the formulae (II) and (III) and acid addition salts and salted esters thereof, which process comprises the reaction of a compounds of the formula (X):

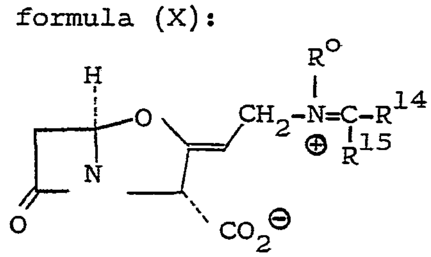

(IX)

or an acid addition salt or salted ester thereof, wherein $R^o$ and $R^{14}$ are as hereinbefore defined and $R^{15}$ is chloro or a group $XR^{13}$ as hereinbefore defined; with a compound of the formula (X):

$$HNR^3R^4 \qquad (X)$$

wherein $R^3$ and $R^4$ are as hereinbefore defined, and thereafter if necessary:

i) removing the ester,
ii) forming an acid addition salt,
iii) forming a salted ester.

The reaction conditions utilised are similar to those for the reaction of the compound of the formula (VI) and the compound of the formula (VII).

In another aspect of this invention there is provided a process for the preparation of the compound of the formula (IV) and acid addition salts and salted esters thereof which process comprises the reaction of a compound of the formula (XI):

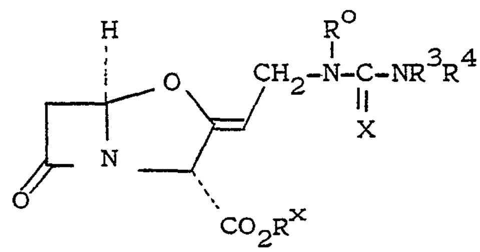

(XI)

wherein $R^o$, $R^x$, $R^3$, $R^4$ and X are as hereinbefore defined, and a reactive alkylating agent, and thereafter if necessary,

i) removing the blocking-group,
ii) forming an acid addition salt,
iii) forming a salted ester.

For reaction wherein X is a sulphuratom suitable alkylating agents include methyl iodide, ethyl iodide, benzyl bromide, dimethylsulphate, allylbromide and methyl chloromethyl ether. Suitable solvents include alcohols, dioxan and acetonitrile at temperature of ambient to 60°C. For reaction wherein X is an oxygen atom suitable alkylating agents include triethyloxonium tetrafluoroborate, trimethyloxonium tetrafluoroborate and methyl fluorosulphonate; suitably at a depressed temperature e.g. -80°C to 0°C in an inert organic solvent.

In a further aspect of this invention there is provided a process for the preparation of a compound of the formula (V) and acid addition salts and salted

esters thereof, which process comprises the reaction of a compound of the formula (XII):

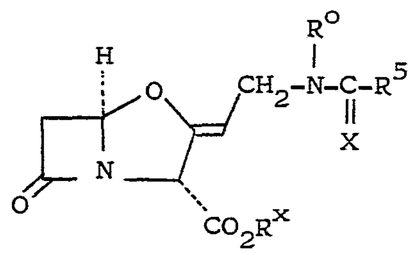

(XII)

wherein $R^o$, $R^x$, X, and $R^5$ are as hereinbefore defined with a reactive alkylating agent, and thereafter if necessary;

i) removing the blocking group,
ii) forming an acid addition salt,
iii) forming a salted ester.

Suitable alkylating agents include triethyloxonium tetrafluoroborate, trimethyloxonium tetrafluoroborate, dimethyl sulphate and methyl fluorosulphonate. Suitably the reaction is performed at a depressed temperature for example -80°C to 0°C, in inert organic solvents such as dichloromethane, chloroform, tetrahydrofuran and dioxan.

Suitable carboxyl-blocking derivatives for the group $-CO_2R^x$ in formula (VI), (XI) and (XII) include ester derivatives of the carboxylic acid. The derivative is preferably one which may readily be cleaved at a later stage of the reaction. Suitable ester-forming carboxyl-blocking groups are those which may be removed under

conventional conditions. Such groups for $R^x$ include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluene-dulphonylethyl, methoxymethyl, a silyl, stannyl or phosphorus-containing group, or an in-vivo hydrolysable ester radical such as defined above. It will be appreciated that it is possible that any carboxyl-blocking group present may be converted to another carboxyl-blocking group during the course of the reaction.

The carboxyl group may be regenerated from any of the above esters by usual methods appropriate to the particular $R^x$ group, for example, acid - and base - catalysed hydrolysis, or by enzymically - catalysed hydrolysis, or by hydrogenation.

The compounds of the formulae (XI) and (XI1) may be prepared by the methods of U.K. Patent Application Number 8118108, which is incorporated by reference.

The compounds of the formula (IX) wherein $R^{15}$ is chloro may be prepared via the reaction of a compound of the formula (XII ) with a halogenating agent such as phosphorus pentachloride, thionyl chloride, phosgene, phosphorustrichloride or phosphorusoxychloride; in the

presence of a base such as triethylamine, pyridine or N-methylmorpholine.

The compounds of the formula (IX) wherein $R^{15}$ is a group $XR^{12}$ may be prepared by the reaction of a compound of the formula (IX) wherein $R^{15}$ is chloro with a $C_{1-6}$ alcohol or $C_{1-6}$ alkylmercaptan in the presence of a base such as triethylamine, pyridine or N-methylmorpholine.

The compounds of the formula (IX) are novel and as such from part of this invention.

A salted ester of a compound of the formula (I) may be prepared by the reaction of the compound of the formula (I) with an esterifying agent. The zwitterionic compound of the formula (I) may be dissolved or suspended in a solvent such as dimethylformamide, hexamethylphosphoramide, dichloromethane, ethyl acetate or other non-esterifiable solvents and therein esterified. Suitable temperatures for such a reaction range from about $0^{o}C$ to about $25^{o}C$. Suitable esterifying reagents include reactive halides and their equivalents and alkyl oxonium salts. When a reagent such as a reactive iodide, chloride, tosylate, mesylate, bromide or equivalent is used, the resulting salt is generally suitable for use in a composition of this invention. Alternatively, the salt may be converted to an alternative salt, for example it is preferable to convert a tetrafluoroborate salt to an alternative salt, for example by passing a solution of one salt through a bed of anion exchange resin in the form of the desired salt, such as the chloride form.

The salts may normally be obtained in solid form by dissolving in a fairly polar organic solvent (such as ethanol or tetrahydrofuran) and then precipitating using a less-polar solvent such as diethyl ether or cyclohexane.

The salted esters of the compounds of the formula (I) may normally be obtained in crystalline form by conventional methods such as trituration under (or crystallisation or recrystallisation form) a suitable organic solvent such as ether, acetone, acetonitrile or tetrahydrofuran.

Salted esters of the compound of the formula (I) may also be prepared by reaction of an acid addition salt of the compound of the formula (I) with an alcohol in the presence of a condensation promoting agent.

Suitable condensation promoting agents for use in this process include carbodiimides such as dicyclohexyl-carboxiimide and the chemical equivalents thereof.

The acid addition salt may be formed in situ or may be preformed. The acid employed will normally be a strong acid such as a methane sulphonic acid, p-toluene sulphonic acid or trifluoroacetic acid.

The reaction is normally carried out in an inert organic solvent. When the ester being formed is that of

a liquid alcohol it is convenient to use that alcohol as the solvent or as part of the solvent system. The esterification is generally performed at a non-extreme temperature such as $0^{o}$ to $35^{o}C$, for example from about $10^{o}$ to $25^{o}C$. Conveniently the reaction may be performed at ambient temperature.

The following Examples illustrates the invention.

EXAMPLE 1

9-N-Benzyl-N-(1-aminoethylidene)ammonio-9-deoxyclavulanate

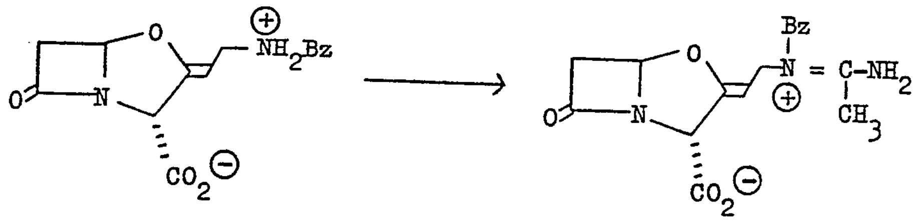

A suspension of 9-N-benzylamino-9-deoxyclavulanic acid (1.0 g, 3.5 mmol) in dry $CH_2Cl_2$ (25 ml) was stirred at $0^o$ and treated with DBN (0.50 g, 4.0 mmol). The resulting solution was treated with ethyl acetimidate hydrochloride (0.43 g, 3.5 mmol) whereupon a heavy precipitate formed. The suspension was refluxed for 8 hrs during which time the precipitate redissolved.

The solvent was removed under reduced pressure and the residue chromatographed on silica gel, eluting with $Bu^nOH/EtOH/H_2O$ (4:1:1) to afford recovered starting material (0.64 g, 64%). Continued elution gave the desired amidine as a white solid (146 mg, 13%).

$\nu_{max}$ (KBr) 1790, 1680, 1620 $cm^{-1}$ $\delta$ ($D_2O$ + DMSO $d_6$) 2.37 and 2.45 (3H, s, C$\underline{H}_3$), 2.82 and 2.88 (1H, d, $\underline{J}$ 17Hz, β-lactam C$\underline{H}$H), 3.50 (1H, dd, $\underline{J}$ 17 and 3Hz, β-

lactam CH*H*), 4.06 and 4.16 (2H, overlapping d, *J* 7Hz, C(9)*H*), ca. 4.6 (2H, s, $NC\underline{H}_2Ph$, obscured by HOD), ca. 4.6 (1H, t, vinyl *H*, obscured by HOD), 4.80 (1H, s, C(3)*H*), 5.62 and 5.64 (1H, overlapping d, *J* 3Hz, β-lactam C*H*), 7.25-7.45 (5H, m, aryl *H*) ppm. Isomer ratio 1:1.

EXAMPLE 2

9-N-Benzyl-N-(aminomethylene)ammonio-9-deoxyclavulanate

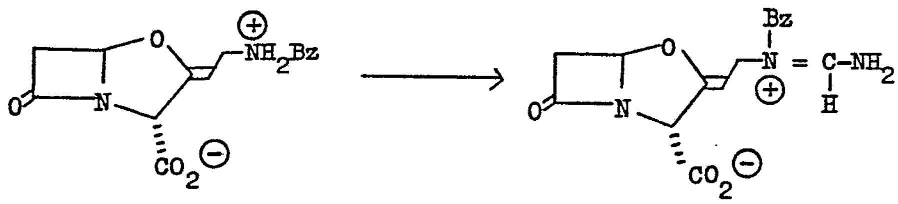

A suspension of 9-N-benzylamino-9-deoxyclavulanic acid (1.0 g, 3.5 mmol) in dry $CH_2Cl_2$ (25 ml) was stirred at $0^o$ and treated with DBN (0.5 g, 4.0 mmol) followed by ethyl formimidate hydrochloride (0.39 g, 3.5 mmol) and the resulting solution refluxed for 18 hrs.

The solvent was evaporated and the residue chromatographed on silica gel. Elution with $Bu^nOH$/EtOH/$H_2O$ (4:1:1) gave recovered starting material followed by the desired product as a white solid (50 mg, 5%).

$\nu_{max}$ (KBr) 1790, 1690, 1600 $cm^{-1}$. $\delta$ ($D_2O$) 2.89 (1H, d, J 17Hz, β-lactam CHH), 3.48 (1H, dd, J 17 and 3Hz, β-lactam CHH), 3.95 and 4.08 (2H, overlapping d, C(9)H), ca. 4.6 (2H, s, $NCH_2Ph$, obscured by HOD), ca. 4.6 (1H, t, vinyl H, obscured by HOD), 4.77 (1H, s, C(3)H), 5.58 and 5.60 (1H, overlapping d, J 3Hz, β-lactam CH), 7.25-7.45 (5H, m, aryl H), 8.03 (1H, s, N = CH) ppm. Isomer ratio ca. 1:1.

Example 3

9-[N-isobutyl-N-(dimethylaminomethylene)ammonio]-9-deoxyclavulanate

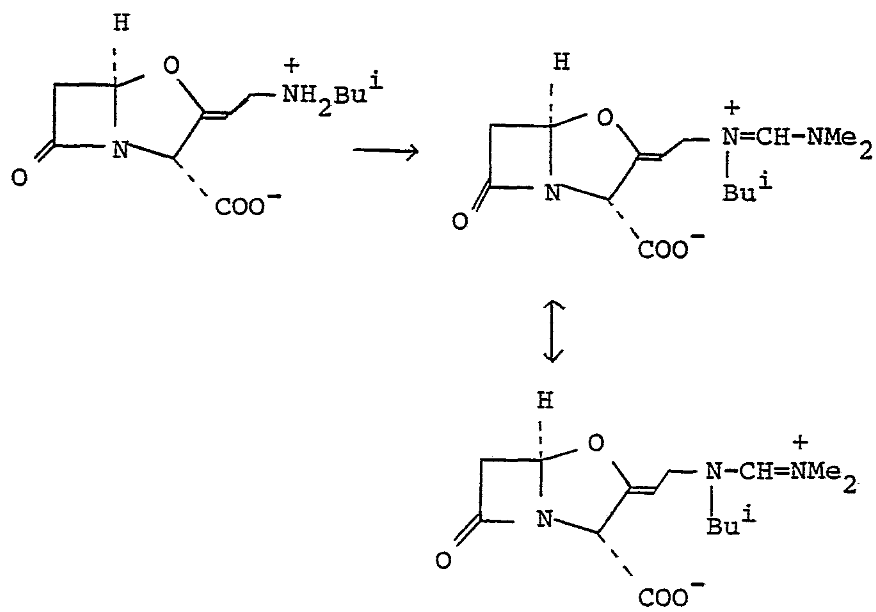

Solid triethyloxonium tetrafluoroborate (900mg) was dissolved in dry dimethylformamide (10ml) and the solution kept 24 hours at room temperature.

9-Isobutylamino-9-deoxyclavulanate (800mg) was suspended in dry dimethylformamide (8ml), the mixture cooled in an ice bath and treated with DBN (0.59 ml). When a complete solution had been obtained, the above mentioned solution (containing N,N-dimethyl-N-ethoxymethylene ammonium fluoroborate) was added dropwise. The resulting solution was stirred for 4 hours at 5-20$^{o}$C, stored overnight in a fridge, treated with water (1 ml) and evaporated (reduced pressure, water bath at

$30^{o}C$) almost to dryness. The residue was chromatographed on silica, eluting with n-butanol/ethanol/water (1.5:1:1). Fractions containing the most polar major β-lactam containing product were combined and evaporated at reduced pressure to near-dryness. The chromatographic procedure was then repeated and appropriate fractions evaporated as before. The residue was dissolved in water (20 ml) and the solutions evaporated at reduced pressure to near-dryness. AR acetone (20 ml) was then added to the residue and evaporated at reduced pressure. This last procedure was repeated (about 6 times) until a foam remained. The residue was desiccated over $P_2O_5$ to provide 9-[N-isobutyl-N-(dimethylaminomethylene) ammonio]-9-deoxyclavulanate (0.41g) as a pale yellow, hygroscopic foam. $\nu_{max}$(KBr) 1785, 1680 and 1620cm$^{-1}$; $\delta$ ($D_2O$:HOD=4.615) 0.84 (6H, d, *J* 7Hz), 1.7-2.4 (1H, m), 2.9-3.4 (9H, m), 3.55 (1H, dd, *J* 17 and 2.5Hz), 4.21 (2H, d, *J* 7.5Hz), 4.79 (1H, t, *J* 7.5Hz), 4.92 (1H, s), 5.71 (1H, d, *J* 2.5Hz), 7.57 (1H, s); $\lambda_{max}$($H_2O$) 222nm ($\epsilon$ 12070).

Biological Data

In a standard MIC synergy test, the following data were obtained for the compounds of this invention:-

| β-Lactamase Inhibitor | | | MIC of amoxycillin (μg/ml) | | |
|---|---|---|---|---|---|
| Testing No. | Example No. | μg/ml | Kleb. E70 | Proteus C889 | E. coli JT39 |
| LI 28745 | 1 | 5<br>1 | 3.1<br>25 | 8<br>31 | 8<br>16 |
| LI 34163 | 2 | 5<br>1 | 3.1<br>6.25 | 31.2<br>125 | 2<br>16 |
| LI 35891 | 3 | 5<br>1 | 6.25<br>25 | 8<br>31.2 | 8<br>125 |
| None | - | - | 500 | >2000 | 2000 |

CLAIMS :

1. A compound of formula (I):

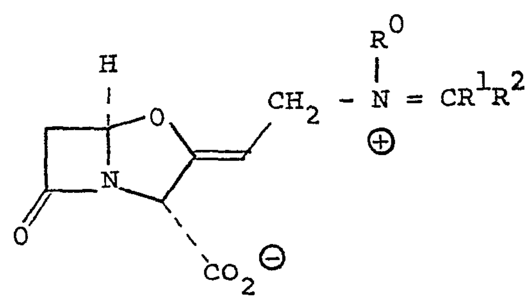

(I)

and acid addition salts and salted esters thereof; wherein $R^0$ is an optionally substituted $C_{1-12}$ hydrocarbon group;

$R^1$ is a group $NR^3R^4$;

$R^2$ is hydrogen or a group R, or $NR^5R^6$;

R is an optionally substituted hydrocarbon or heterocyclic group;

$R^3$ is hydrogen or a group R or OR;

$R^4$, $R^5$ and $R^6$ are independently hydrogen or a group R; or

$R^3$ and $R^4$, $R^5$ and $R^6$, or $R^1$ and $R^2$ may be joined to form a heterocyclic ring.

2. A compound as claimed in claim 1 wherein $R^O$ represents $C_{1-6}$alkyl or benzyl.

3. A compound as claimed in either claim 1 or claim 2 having the formula (II) :

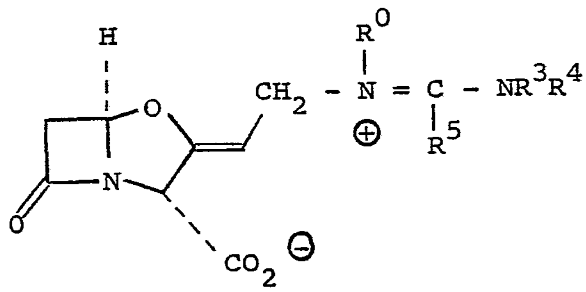

(II)

and acid addition salts and salted esters thereof, wherein $R^3$, $R^4$ and $R^5$ are as defined in claim 1 and $R^o$ is as defined in either claim 1 or claim 2.

4. A compound as claimed in claim 3 wherein $R^5$ represents hydrogen or $C_{1-6}$ alkyl.

5. A compound as claimed in claim 3 or 4 wherein $R^3$ and $R^4$ independently represent hydrogen or $C_{1-6}$ alkyl.

6. A process for the preparation of a compound of formulae (II) and (III) and acid addition salts and salted esters thereof, which process comprises the reaction of a compound of the formula (VI) or a derivative thereof that allows alkylation to occur:

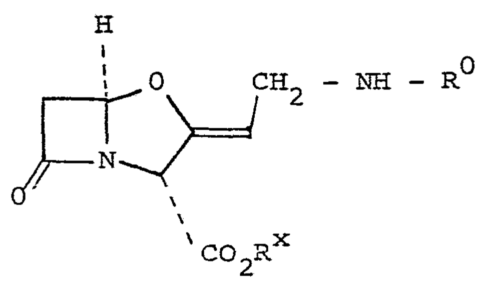

(VI)

wherein $R^0$ is as hereinbefore defined and $R^x$ is a hydrogen atom or a carboxy-blocking group; with either the compound of the formula (VII) or (VII):

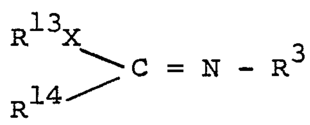

(VII)

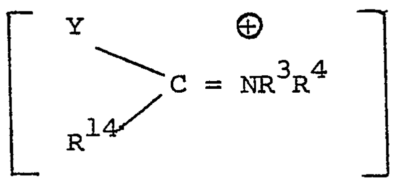

(VII)

wherein $R^{13}$ is $C_{1-6}$ alkyl, $R^{14}$ is a group $R^5$ or $NR^5R^6$ wherein $R^5$ and $R^6$ are as hereinbefore defined, Y is halo or $C_{1-6}$ alkoxy, X is an oxygen or sulphur atom, and $Z^-$ is a salting-ion, and $R^3$ and $R^4$ are as defined hereinbefore; and thereafter if necessary

i) removing the blocking group,

ii) forming an acid addition salt,

iii) forming a salted ester.

7. A process for the preparation of a compound of formulae (II) and (III) and acid addition salts and salted esters thereof, which process comprises the reaction of a compounds of the formula (X):

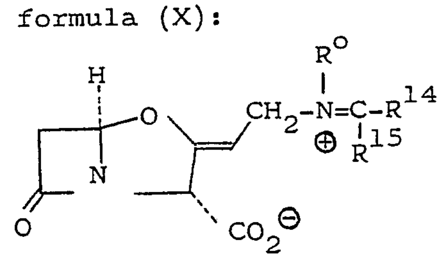

(IX)

or an acid addition salt or salted ester thereof, wherein $R^o$ and $R^{14}$ are as hereinbefore defined and $R^{15}$ is chloro or a group $XR^{13}$ as hereinbefore defined; with a compound of the formula (X):

$$HNR^3R^4 \qquad (X)$$

wherein $R^3$ and $R^4$ are as hereinbefore defined, and thereafter if necessary:

i) removing the ester,
ii) forming an acid addition salt,
iii) forming a salted ester.

8. A pharmaceutical composition comprising a compound as claimed in claim 1 together with a pharmaceutically acceptable carrier.

9. A composition as claimed in claim 8 which further comprises a β-lactam antibiotic.

10. A compound as claimed in claim 1 for use as a β-lactamase inhibitor or antibacterial agent.

0068715

European Patent Office

# EUROPEAN SEARCH REPORT

Application number

EP 82 30 3112

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP-A-0 010 369 (BEECHAM) *Page 21* | 1,6 | C 07 D 498/04<br>A 61 K 31/42 //<br>(C 07 D 498/04<br>C 07 D 263/00<br>C 07 D 205/00 )<br>(A 61 K 31/42<br>A 61 K 31/43 )<br>(A 61 K 31/42<br>A 61 K 31/545) |
| | --- | | |
| P,X | EP-A-0 046 349 (BEECHAM) *Claims* | 1-10 | |
| | ----- | | |

| TECHNICAL FIELDS SEARCHED (Int. Cl. 3) |
|---|
| C 07 D 498/00<br>A 61 K 31/00 |

The present search report has been drawn up for all claims

| Place of search | Date of completion of the search | Examiner |
|---|---|---|
| THE HAGUE | 16-09-1982 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82